# EUROPEAN PATENT APPLICATION

(11) **EP 2 163 647 A1**
(43) Date of publication of application: **17.03.2010**
(21) Application number: 08016221.7
(22) Date of filing: 15.09.2008
(51) Int. Cl.: C12Q 1/68

(54) **Method to eliminate polymerase chain reaction inhibitors from feacal samples**

(71) Applicant: Serosep Limited, Limerick (IE)
(72) Inventor: Russell, Tom, Co Limerick (IE); Scanlon, Dermot, Co Limerick (IE); Molnar, Camelia, Turda, 401023, Cluj (RO)
(74) Representative: Schneider, Michael

(57) **Abstract**

The present invention relates to the field of sample preparation methods and kits for PCR diagnosis of gastroenteritis. Disclosed is a method for removing polymerase chain reaction (PCR) inhibitory substances from faecal samples and disrupting bacterial cells to be assayed prior to PCR by using a two-phase aqueous polymer system and by subjecting the cells to ultrasonic energy.

## Description

The present invention relates to the field of sample preparation methods and kits for PCR diagnosis of gastroenteritis.

A method is disclosed for removing of polymerase chain reaction (PCR) inhibitory substances from faecal samples and disrupting bacterial cells prior to PCR, by using a two-phase aqueous polymer system and by subjecting the cells to ultrasonic energy.

### Background of the invention

Gastroenteritis is still a major health problem worldwide. When stool culture is requested, the specimen will be examined routinely for *Salmonella* spp., *Shigella* spp., *Campylobacter* spp. and *Escherichia coli* O157. It is still a challenge to detect these pathogens fast and accurately from clinical samples. Conventional methods for the detection and identification of these enteric pathogens include growth on specific selective media, followed by biochemical and serological confirmation using commercial available kits. The limitation of the conventional methods is that they are time consuming and labour intensive. Presumptive results are available only after two days. Molecular methods have become very rapid tools for the diagnosis of a variety of infectious diseases. The molecular techniques can overcome the problems of the conventional methods. There are a number of previously published rapid methods for the detection of the enteric pathogens in stool samples, but most of these methods are not easily applicable in routine diagnosis in the clinical laboratory, because they use expensive equipment, labour intensive DNA extraction methods or use harmful chemicals.

The presence of enzyme-inhibitors in stool samples makes the use of rapid molecular detection methods using enzymatic amplification very difficult. The success of the molecular techniques on clinical samples depends on the use of appropriate DNA extraction kits or methods.

Polymerase chain reaction (PCR) based assays are sensitive and specific tools for the detection of microorganisms in different samples. The successful detection of bacterial DNA demands efficient extraction of DNA from the samples as incomplete disruption of cellular structure leads to incomplete release of DNA. The efficiency of the lysis is therefore a factor that can limit the sensitivity of the PCR assay. There is a need as well for a simple way to remove the PCR inhibitors from stool samples and to break bacterial cells in an efficient manner suitable for the clinical laboratory.

### Description of the prior art

US patent 4,980,065 granted to Hsu relates to a two-phase system on the basis of a water soluble polymer such as polyethylene glycol and a chiral compound such as peptides useful for the separation and purification of biochemicals and optical isomers. Application of aqueous two-phase systems for the separation of macromolecules, cells, viruses or organelles from a mixture by their distribution between two immiscible solutions has been proven useful by Albertsson (Partition of Cell Particles and Macromolecules, 3rd ed. Wiley, New York, 1986). Aqueous two-phase systems are biphasic systems obtained by blending two polymer aqueous solutions or a polymer solution and a salt solution. The polyethylene glycol (PEG)-dextran system is the most extensively studied. Lantz et al. (J. Microbiol. Meth. 28 (1997), pp. 159-167) demonstrated the efficiency of an aqueous two-phase system, composed of 8% (w/w) PEG 4000 and 11% (w/w) dextran 40, for the separation of *Helicobacter pylori* and PCR inhibitors originating from faeces. Their method involved centrifugation for 1 minute at 100 x g of homogenized human faecal samples in water to remove visible debris, and heating of the supernatant in a boiling waterbath for 10 minutes. 100µl of boiled supernatant was then employed as the sample in the aqueous two-phase system. The system was inverted 20 times and then allowed to separate by gravity settling for 60 minutes at room temperature. Samples were then withdrawn from the bottom phases and subjected to PCR. The disadvantages of this method are that it is difficulty to implement routinely in a clinical laboratory, that the demixing under gravity is very slow, and that the detection level of *H. pylori* cells needs to be improved by concentration of *H. pylori* cells using an enrichment step and by a lysis step to make the DNA target available for PCR.

Murphy et al. (1994), US Patent 5,374,522, discovered that cells in a solution with small beads (glass, plastic, sand or silicates, latex, crystals, metals) in a container, when subjected to sonication, released their DNA into solution within minutes. Later, Llorin et al. (2005), US patent 6,939,696, describe a method for lysing cells wherein a sample containing cells is subjected to ultrasonic energy without the presence of beads. Following such lysis, nucleic acid from the cells is available for use in various molecular biology procedures. They have found the optimal factors for the lysis of *Mycobacterium tuberculosis* to be extreme alkaline conditions, for 30 minutes, the temperature of the waterbath above 65°C and when frequency of 35 kHz of ultrasonic treatment is used.

Raghavarao et al. (2000), US Patent 6,090,295, describe a method for demixing aqueous solutions, which reduces the demixing time by application of acoustic energy to the aqueous solution.

From the above disadvantages of the prior art and the problems involved therewith the objects of the present invention become apparent.

### Summary of the invention

The object of the present invention is to provide a method to remove PCR inhibitories from stool samples and to lyse the bacterial cells making the DNA target available for PCR, in a short period of time and suitable to be used routinely in clinical laboratories.

This object is accomplished by the present invention by providing a method to remove PCR inhibitories and to disrupt bacterial cells as defined in present claim 1.

In one embodiment the process generally includes the steps of: centrifugation of the enriched faecal samples; transfer of the supernatant to an aqueous two phase system, mixing the sample with the aqueous two phase system and sonicating the mix into a waterbath sonicator for 30 minutes. Following the ultrasonic lysis, nucleic acids from the bacterial cells are available for use from the bottom phase of the system for PCR-analysis with no further purification.

In a further aspect the invention provides a kit for application of the inventive method in a faecal pathogen detection assay as defined in present claim 13.

Preferred and/or advantageous embodiments of the invention are subject-matter of the subclaims.

In the following the invention is described in more detail with reference to examples. These examples are intended for illustration only and are not to be construed as any limitation.

### Detailed description of the invention

In this work the potential of aqueous polymer two-phase system for the separation of enteric pathogens from complex stool samples has been demonstrated.

The inventive method is based on the use of a two-phase aqueous polymer system, PEG/Dextran mix, where PEG and water are forming the upper phase and dextran, salt and water are forming the bottom phase. As salts are used sodium phosphates to increase the tonicity of the solution and to adjust the pH. The aqueous two-phase system is prepared from the stock solutions of the polymers.

The aqueous two-phase system was proven to be an excellent method for the separation of enteric pathogens and PCR inhibitors originating from faeces (bile acids: cholic acid, deoxycholic acid). The aqueous two-phase system is employed to separate PCR inhibitories from bacterial cells. *Campylobacter* spp., *Shigella* spp., *E*. *coli* O157, *Salmonella* spp. are found in the bottom phase, while the PCR inhibitory bile acids are found in the top phase, resulting in a bottom phase with a low concentration of bile acids. As the test ubes remain closed during the sonication, there is no risk of cross-contamination. The method avoids the use of toxic and explosive components (phenol, ether). It is performed in 30 minutes using a single tube with two simple steps.

The rationale for using sonication is to achieve complete disruption of cellular structures and release of DNA without the need for lysing reagents and time-consuming sample preparation. The necessary separation of the two immiscible liquid phases, which is relatively slow under gravity, is enhanced using sonication. The simplicity of the method results from the limited handling of a sample and minimal additions to the sample.

In the present process the target is partitioning mainly to the bottom phase, while most of the contaminants are separating mainly to the top phase. To release the DNA from the cells, ultrasonic treatment was done, for example, by immersing the 8ml tubes of the PEG/Dextran system containing 200µl enriched faecal samples in a waterbath type sonicator (Elma Transsonic Digital) at a temperature above 65°C, 50mW/cm³ (140% of the power), 40kHz for 30 minutes. Acoustic field assisted demixing was employed as well to decrease the demixing time in polymer-polymer two phase system. Ultrasonication of the aqueous two-phase system causes mild circulation currents in the phase dispersion, and enhances the rate of droplet coalescence, resulting in a decreased demixing time. For an effective sonication processing, any gas bubbles must be removed from the liquid as they could absorb energy and affect the results. The degassing is normally done by running the device for a few minutes until free bubbling ceases.

A variety of ultrasonic baths are commercially available and useful for the present invention. Examples of suitable ultrasonic baths include those available from Elma, Germany, which markets a number of models under the Elmasonic name with tank capacities ranging from 0.9 to 90 litres, an operating frequencies of about 37 to about 40 kHz and a temperature range from ambient to about 90°C.

The claimed method for removing polymerase chain reaction (PCR) inhibitory substances from faecal samples and disrupting target bacterial cells in said samples prior to PCR assaying said cells comprises the steps of:
(a) mixing a faecal sample containing target bacterial cells to be assayed by PCR with a two-phase aqueous polymer system composed of 8% (w/w) polyethylene glycol 4000 (PEG 4K) and 11 % (w/w) dextran 40;
(b) subjecting the mixture obtained in step (a) to ultrasonic energy of sufficient power and duration to cause disruption of said bacterials and to cause enhanced separation of the two phases; and
(c) separating the dextran-rich bottom phase for PCR assaying for target bacterials.

It is appreciated that the exact concentrations for PEG 4K and dextran 40 composing the two-phase system are of no critical importance to achieve the extraction effect of the inventive method. Other concentrations can easily be found by trial and error measuring the partition concentration of the PCR inhibitory bile acids in the top and bottom phases. The same is true for other two-phase aqueous polymer systems not based on PEG and dextran.

The used power and duration of the applied ultrasonic energy is also of no critical importance as long as disruption of bacterial cells and enhanced separation of the two phases is achieved without destruction of the ability of the nucleic acids to be liberated and extracted to bind (hybridize) to a complementary nucleic acid molecule (such as a PCR primer or any genetic probes) compared to an intact nucleic acid molecule.

In one embodiment of the inventive method the faecal sample is prior to the above step (a) homogenized in water or in a suitable buffer, such as in commonly used phosphate buffers or in Tris-buffered saline, (pH range about 6 to 12, e.g. about 7 or about 8), and removed, e.g. by centrifugation (for example at about 100 x g for about 1 min) from visible material such as debries to obtain a clear sample. The faecal sample may advantageously be degassed prior to step (a) according to established methods, for example by heating to about 60 °C for 15 to 30 minutes, to avoid bubbling in the sonication step. The defined concentration ranges for PEG 4K and dextran 40, respectively, can easily be adjusted by adding suitably high concentrated stock solutions of PEG 4K and dextran 40 to the sample until, under consideration of the sample volume, the desired final concentration is reached.

In another embodiment of the inventive method the used ultrasonic power density in step (b) is in the range of from 25 to 250 mW/cm³. A preferred ultrasonic power density is about 50 mW/cm³, The ultrasonic frequency is in the range of from 25 to 75 KHz. A preferred frequency is about 40 KHz.

In yet another embodiment of the inventive method the ultrasonic frequency used in step (b) varies with time in the range of from 25 to 75 KHz, i.e. a sweeping frequency may be used.

In still another embodiment of the inventive method the faecal sample in step (a) is heated to a temperature in the range of from ambient to 90 °C, for example in the range of from 60 °C to 85 °C. A preferred temperature is about 65°C. For example, for degassing the temperature of the faecal sample may be raised to about 60°C. By sonication the sample at about 50 mW/cm³ and about 40 KHz for about 30 min, for example, the temperature may increase further to about 85°C.

In another embodiment of the inventive method the ultrasonic energy is in step (b) applied to the faecal sample for a time in the range of from 20 to 60 minutes. A preferred application time is 30 minutes

It is appreciated that a shorter application time may be compensated by a higher ultrasonic energy, and that a lower ultrasonic energy may be compensated by longer application time. In general, shorter application times are preferred, and a suitable combination of application time and ultrasonic energy for a given faecal sample can easily be determined.

### Example

### Comparison of aqueous two phase system against QiAamp DNA mini kit method

### Equipment and Materials

- Centrifuge capable of 100 x g with 10 ml tubes
- Centrifuge capable of 14.000 rpm for eppendorf tubes
- Waterbath sonicator (Elma Transsonic Digital)
- Sonicator rack
- Thermocycler
- Shaker
- Incubator
- Pipettor (1ml; 200µl; 10µl)
- QiAamp DNA ministool kit
- 20% Dextran 40 solution
- 40% PEG 4000 solution
- 100mM phosphate buffer (pH 7.0)
- Enrichment broth
- Pathogen detection kit
- *E. coli* O157, *Shigella* spp., *Campylobacter* spp., *Salmonella* spp. isolates
- Stool samples

We evaluated the aqueous two-phase system and a commercially available kit QiAamp DNA ministool kit (Qiagen) for their ability to extract *Salmonella* spp., *E*. *coli* O157, *Campylobacter* spp. and *Shigella* spp. DNA from enriched stool specimens for PCR. Sensitivity, processing time, and cost were compared between the different kits. The methods were evaluated by using spiked faecal samples with different dilutions of each of the four pathogens.

### Sample preparation

A panel of human specimens submitted for routine investigation of acute infectious gastroenteritis were included in this study following Institutional Review Board approval to ensure patient confidentiality. All specimens were tested for enteric infections by conventional culture methods. Stools obtained from healthy people were used for spiking experiments. Faecal specimens were stored at -200C until processed. Commercially available enrichment broths designed to simultaneously grow all four pathogens Salmonella spp., E. coli O157, Shigella spp. and Campylobacter spp. were inoculated with spiked faecal samples and then incubated overnight at 37 °C.

Centrifuge the enriched sample at 100 x g for 1 minute.

Use the supernatant for the DNA extraction.

Samples were processed by either of two nucleic acid extraction procedures.

### Aqeous two phase system method

The aqueous two-phase system containing water soluble polymers is prepared as follows:

40% (w/w) PEG stock solution and 20% (w/w) dextran stock solution are made up by accurate weighing of the powder and dissolution in water at room temperature. The stock concentration can be measured by refractive index. The stock solutions are sterilised at 121°C and 1 atmosphere for 15 minutes.The aqueous two-phase system may be prepared from the stock solutions of the polymers. The stock solutions may contain phosphate buffer to control the pH of the solution.

The total weight of the two-phase system is approximately 2.5g out of which 0.2ml is the volume of enriched faecal sample and 0.25ml is the volume of 0.1M phosphate buffer (pH 7.0). Water, 0.1 M phosphate buffer and 40% (w/w) PEG 4000 stock solution are mixed to obtain a single solution that is then mixed with the 20% (w/w) dextran stock solution. 0.2ml of the enriched faecal sample is added at the end to obtain the two-phase system with the final concentrations of 8% (w/w) PEG 4000 and 11% (w/w) dextran.

### Equipment preparation

### Sonicator

For optimal transfer of sonic energy, water must be degassed according to the following procedure:
Fill the ultrasonic bath with boiled water. Activate the degas function of the sonicator for 15 minutes. When the degas function is switched on, the green indicator flashes and the liquid is degassed.

### Sample lysis

1. Add 200µl of the supernatant to the two-phase aqueous polymer system composed of 8% (w/w) polyethylene glycol 4000 and 11 % (w/w) dextran 40. Mix the system by inversion, approximately 20 times.
   Push the two-phase system tube through the Sonicator rack so that the mixture in the bottom of the tube is submerged but the cap is above the water.
   Place the sonicator rack on waterbath sonicator (Elma Transsonic Digital). Do not allow the tubes to touch the bottom or sides of the sonicator.
2. Sonicate for 30 minutes at a temperature above 70° C, 40kHz and 140% of the power.
3. A top phase rich in PCR inhibitors and a bottom phase rich in bacterial DNA should form.
4. Withdraw 10µl sample from the bottom phase to be used in the PCR reaction (PCR reaction volume 25µl).

### QiAamp DNA stool mini kit purification method

The QiAamp DNA purification procedure is based on digestion of proteins with proteinase K, binding DNA to a silica-gel column, washing out the impurities and elution of pure DNA from the membrane of column. The extraction with the QiAamp DNA stool minikit were performed in accordance with the manufacturer's instructions using the protocol for isolation of DNA from stool for pathogen detection. The lysis of the specimens was performed at 95°C instead at 70°C. The volume of eluate used as template (2.5µl) was not exceed 10% of the final volume of the PCR mixture.

### PCR assay

A commercially available faecal pathogen detection kit for the detection of Salmonella spp., E. coli O157, Shigella spp. and Campylobacter spp. based on standard molecular biology methods is used. Genes that are idiosyncratic, respectively, for the genus Salmonella, E. coli O157, the genus Shigella and the genus Campylobacter, and an internal positive control are amplified in a single reaction (multiplex PCR) and detected afterwards by sandwich hybridization and color development.

PCR assay was performed according with the instructions provided in the pack insert.

### Results

**Detection of enteric pathogens by PCR assay in stool samples inoculated with various concentrations of the organism using the Qiangen kit and aqueous two phase system**

| **CFU/g** | ***E.coli O157*** | | ***Shigella spp*** | | ***Salmonella spp*** | | ***Campylobacter*** | |
|---|---|---|---|---|---|---|---|---|
| | ATPS^{*} | Qiagen | ATPS | Qiagen | ATPS | Qiagen | ATPS | Qiagen |
| 10⁸ | ++ | ++ | ++ | ++ | ++ | ++ | ++ | ++ |
| 10⁷ | ++ | ++ | ++ | ++ | ++ | ++ | ++ | ++ |
| 10⁶ | ++ | ++ | ++ | ++ | ++ | ++ | ++ | ++ |
| 10⁵ | ++ | ++ | ++ | ++ | ++ | ++ | ++ | ++ |
| 10⁴ | ++ | ++ | ++ | ++ | ++ | ++ | ++ | -- |
| 10³ | ++ | ++ | ++ | +- | ++ | ++ | -- | -- |
| 10² | ++ | ++ | ++ | -- | ++ | ++ | - | -- |
| 10¹ | ++ | ++ | +- | -- | ++ | ++ | - | -- |
| 10⁰ | +- | +- | -- | -- | ++ | -+ | - | -- |

| | | | | | | | | |
|---|---|---|---|---|---|---|---|---|
| ATPS- aqueous two phase system method | | | | | | | | |

The purpose of the addition of known bacterial colony forming units to each test faecal sample was to provide a minimum and a serially increasing number of known bacteria to asses the relative performance of the two DNA extraction methods. None of the PCRs with spiked samples were inhibited, as each samples amplified the PCR internal control as well.

In the present example, the aqueous solution system was demixed to clarity in approximately 30 minutes. Without the application of the acoustic energy, the aqueous solution system was demixed to clarity in approximately one hour.

We have shown that different extraction techniques result in variable sensitivity of PCR detection for DNA of enteric pathogens in human faecal samples.
The inventive aqueous two phase system method was the most sensitive procedure for the extraction of DNA of enteric pathogens from stool samples and led to the detection of bacterial DNA over the greatest range of spike concentrations using PCR assay.

## Claims

1. A method for removing polymerase chain reaction (PCR) inhibitory substances from faecal samples and disrupting target bacterial cells in said samples prior to PCR assaying said cells comprising the steps of:
(a) mixing a faecal sample containing bacterial cells to be assayed by PCR with a two-phase aqueous polymer system composed of 8% (w/w) polyethylene glycol 4000 and 11 % (w/w) dextran 40;
(b) subjecting the mixture obtained in step (a) to ultrasonic energy of sufficient power and duration to cause disruption of said bacterials and to cause enhanced separation of the two phases; and
(c) separating the dextran-rich bottom phase for PCR assaying for target bacterials.

2. The method according to claim 1, wherein prior to step (a) the faecal sample is homogenized in water or buffer and removed from visible material to obtain a clear sample.

3. The method according to claim 2, wherein the visible material is removed by centrifugation.

4. The method according to anyone of the preceding claims, wherein the faecal sample is degassed prior to step (a).

5. The method according to anyone of the preceding claims, wherein in step (b) the ultrasonic power density is in the range of from 25 to 250 mW/cm³ and the ultrasonic frequency is in the range of from 25 to 75 KHz.

6. The method according to claim 5, wherein the ultrasonic power density is 50 mW/cm³ and the frequency is about 40 KHz.

7. The method according to claim 5, wherein the ultrasonic frequency varies with time in the range of from 25 to 75 KHz.

8. The method according to anyone of the preceding claims, wherein in step (a) the faecal sample is heated to a temperature in the range of from ambient to 90°C.

9. The method according to claim 8, wherein the temperature in the range of from 60°C to 85°C.

10. The method according to claim 9, wherein the temperature is about 65°C.

11. The method according to anyone of the preceding claims, wherein in step (b) the ultrasonic energy is applied for a time in the range of from 20 to 60 minutes.

12. The method according to claim 11, wherein the ultrasonic energy is applied for about 30 minutes.

13. A kit for application of the method according to anyone of claims 1 to 12 in a faecal pathogen detection assay comprising a tube with a two-phase aqueous polymer system composed of 8% (w/w) polyethylene glycol 4000 and 11 % (w/w) dextran 40, an internal positive control, and polymerase chain reaction regents for specifically detecting the faecal pathogens.

14. The kit according to claim 13 further comprising an enrichment broth for the faecal pathogens.

15. The kit according to claims 13 or 14, wherein the polymerase chain reaction reagents comprise a thermostable DNA polymerase, at least two specific oligonucleotide primers for each pathogen to be detected, buffer and nucleotides.

16. The kit according to anyone of claims 13 to 15, wherein the faecal pathogens are selected from the group consisting of the genus Salmonella, E. coli 0157, the genus Shigella and the genus Campylobacter.

## Amended claims

### Amended claims in accordance with Rule 137(2) EPC.

**1.** A method for removing polymerase chain reaction (PCR) inhibitory substances from faecal samples and disrupting target bacterial cells in said samples prior to PCR assaying said cells comprising the steps of:
(a) mixing a faecal sample containing bacterial cells to be assayed by PCR with a two-phase aqueous polymer system composed of 8% (w/w) polyethylene glycol 4000 and 11 % (w/w) dextran 40;
(b) subjecting the mixture obtained in step (a) to ultrasonic energy of sufficient power and duration to cause disruption of said bacterials and to cause enhanced separation of the two phases; and
(c) separating the dextran-rich bottom phase for PCR assaying for target bacterials.

**2.** The method according to claim 1, wherein prior to step (a) the faecal sample is homogenized in water or buffer and removed from visible material to obtain a clear sample.

**3.** The method according to claim 2, wherein the visible material is removed by centrifugation.

**4.** The method according to anyone of the preceding claims, wherein the faecal sample is degassed prior to step (a).

**5.** The method according to anyone of the preceding claims, wherein in step (b) the ultrasonic power density is in the range of from 25 to 250 mW/cm³ and the ultrasonic frequency is in the range of from 25 to 75 KHz.

**6.** The method according to claim 5, wherein the ultrasonic power density is 50 mW/cm³ and the frequency is about 40 KHz.

**7.** The method according to claim 5, wherein the ultrasonic frequency varies with time in the range of from 25 to 75 KHz.

**8.** The method according to anyone of the preceding claims, wherein in step (a) the faecal sample is heated to a temperature in the range of from ambient to 90°C.

**9.** The method according to claim 8, wherein the temperature in the range of from 60°C to 85°C.

**10.** The method according to claim 9, wherein the temperature is about 65°C.

**11.** The method according to anyone of the preceding claims, wherein in step (b) the ultrasonic energy is applied for a time in the range of from 20 to 60 minutes.

**12.** The method according to claim 11, wherein the ultrasonic energy is applied for about 30 minutes.
